# EUROPEAN PATENT SPECIFICATION

(11) **EP 2 140 882 B1**
(45) Date of publication and mention of the grant of the patent: **14.08.2013**
(21) Application number: 08751606.8
(22) Date of filing: 25.04.2008
(51) Int. Cl.: A61K 45/00, A61K 9/51, A61K 31/22, A61K 31/366, A61K 31/40, A61K 31/405, A61K 31/47, A61K 31/505, A61K 47/34, A61P 11/00

(54) **AGENT FOR TREATMENT OF PULMONARY DISEASE**
MITTEL ZUR BEHANDLUNG VON LUNGENERKRANKUNGEN
AGENT POUR LE TRAITEMENT D'UNE MALADIE PULMONAIRE

(30) Priority: 27.04.2007 JP 2007119553; 13.06.2007 JP 2007155816
(43) Date of publication of application: 06.01.2010
(73) Proprietor: Kyushu University, National University Corporation, Fukuoka-shi Fukuoka 812-8581 (JP); Kowa Co., Ltd., Nagoya-shi, Aichi 460-8625 (JP)
(72) Inventor: EGASHIRA, Kensuke, Fukuoka-shi Fukuoka 812-8581 (JP); KOJIMA, Junji, Higashimurayama-shi Tokyo 189-0022 (JP); SAKAMOTO, Megumi, Higashimurayama-shi Tokyo 189-0022 (JP)
(74) Representative: Blodig, Wolfgang
(86) International application number: PCT/JP2008/001081
(87) International publication number: WO 2008/139703

(56) References cited:
- WO-A1-03/103640
- WO-A1-2008/040967
- WO-A2-00/48626
- JP-A- 2004 521 152
- US-A1- 2005 119 330
- KUBO M ET AL: "A Novel Nanotechnology-Based Strategy for Therapeutic Neovascularization: Local Delivery of Statin with Biodegradable-Polymeric-Nanoparticle Improves Therapeutic Efficacy of Ischemic Neovascularization" CIRCULATION JOURNAL, JAPANESE CIRCULATION SOCIETY, KYOTO, JP, vol. 71, no. SUPPL. 1, 1 March 2007 (2007-03-01), page 298, XP003021340 ISSN: 1346-9843
- 'Nanospheres tailored to inhalation devices' MANUFACTURING CHEMIST vol. 77, no. 9, September 2006, pages 75 - 76, 78, XP008119399
- MIZOE T. ET AL.: 'Nano Ryushi Gan'yu Microsphere no Sekkei to DDS eno Oyo' SOCIETY OF POWDER TECHNOLOGY, JAPAN.FUNTAI KOGYO GIJUTSU KYOKAI GIJUTSU TORONKAI.TEXT vol. 41ST, 2006, pages 19 - 20
- POYNER E.A. ET AL.: 'A comparative study on the pulmonary delivery of tobramycin encapsulated into liposomes and PLA microspheres following intravenous and endotracheal delivery' JOURNAL OF CONTROLLED RELEASE vol. 35, no. 1, July 1995, pages 41 - 48, XP004037491
- BANDO Y. ET AL.: 'Choki Kecchu Tairyusei o Mokuteki to shita Seibunkaisei Kobunshi Nanosphere no Sekkei' THE PHARMACEUTICAL SOCIETY OF JAPAN NENKAI YOSHISHU vol. 127TH, no. 3, 05 March 2007, page 126 + ABSTR. NO. 29P1-AM293

## Description

### Technical Field

The present invention relates to the drug pitavastatin for use in the treatment of pulmonary diseases (hereinafter may be referred to as a "pulmonary disease therapeutic drug"), which drug exhibits excellent effects of ameliorating pulmonary diseases.

### Background Art

Intractable pulmonary diseases (e.g., chronic obstructive pulmonary disease (COPD), pulmonary fibrosis, acute respiratory distress syndrome (ARDS), and pulmonary hypertension) cause impairment of QOL and have very poor prognosis. Such an intractable pulmonary disease (e.g., pulmonary hypertension) has a five-year survival rate of 50% or less. Recently, new remedies (e.g., sildenafil, bosentan, and continuous intravenous infusion of prostacyclin) have been used for treatment of severe pulmonary hypertension, but these remedies exhibit unsatisfactory effects. Furthermore, the remedies cannot complete cure, for example, chronic obstructive pulmonary disease or pulmonary fibrosis. Therefore, demand has arisen for research and development of a fundamental and low-invasive therapeutic method for severe pulmonary diseases.

Many acute or chronic respiratory diseases (e.g., bronchial asthma, COPD, and pulmonary fibrosis (interstitial pneumonia)) involve pathologic conditions associated with airway inflammation.
As has been known, in a general course of development of inflammatory conditions, firstly, signaling molecules (called "chemotactic factor") released at an early stage promote migration of inflammatory cells (e.g., neutrophils, basophils, eosinophils, and macrophages) to local sites, and the migrating inflammatory cells cause the release of enzymes or radicals which give damage to tissue, and as well release cytokines or similar factors, to thereby further cause migration and activation of inflammatory cells. When such inflammation is developed at the airway, infiltrated inflammatory cells cause damage to bronchial or lung tissue, which eventually results in respiratory dysfunctions characteristic of the aforementioned diseases, such as reduction in respiratory flow rate or oxygen exchange capacity.

On the basis of such findings, drugs exhibiting antiinflammatory effect have been applied to the treatment of inflammatory respiratory diseases. As has been already known, adrenocortical steroid is remarkably effective for mild to moderate bronchial asthma (Non-Patent Document 1). Also, adrenocortical steroid has been reported to prevent exacerbation of COPD. However, adrenocortical steroid exhibits a limited effect on COPD (Non-Patent Document 2). Hitherto, there have not yet been obtained data that positively support the efficacy of adrenocortical steroid on pulmonary fibrosis (Non-Patent Document 3). Meanwhile, adrenocortical steroid has been known not only to non-specifically inhibit immune function, but also to possibly cause various side effects, such as electrolyte abnormality, peptic ulcer, myopathy, behavioral abnormality, cataract, osteoporosis, osteonecrosis, and growth inhibition (Non-Patent Document 4).

With the pervasion of therapies mainly using an inhaled steroid for the treatment of bronchial asthma, the number of emergency outpatients or inpatients with asthmatic attack has decreased, and the number of controllable outpatients has increased.
Even under such circumstances, the number of asthma patients is not reduced, and asthmatic deaths from fatal attack still occur. That is, currently available antiasthmatic combination therapies mainly using an inhaled steroid still do not exhibit satisfactory therapeutic effects. Therefore, demand has arisen for development of a new therapeutic agent having high efficacy and reduced side effects.

In recent years, retrospective clinical studies (epidemiological studies) on HMG-CoA reductase inhibitors, which exhibit a potent LDL-cholesterol lowering effect and are used as drugs of first choice for the treatment of hyperlipidemia, reported that use of HMG-CoA reductase inhibitors contributes to the survival rate of COPD patients (Non-Patent Documents 5 and 6). Other studies previously reported that HMG-CoA reductase inhibitors exhibit an antiinflammatory effect, which is one of pleiotropic effects independent of the LDL-cholesterol lowering effect thereof, and suggested the possibility of application of HMG-CoA reductase inhibitors to the aforementioned inflammatory pulmonary diseases (Non-Patent Document 7).

However, when an HMG-CoA reductase inhibitor is orally administered, the absorbed HMG-CoA reductase inhibitor is accumulated specifically in the liver by the involvement of a drug transporter. Therefore, high-dose administration of an HMG-CoA reductase inhibitor is required for accumulation of the inhibitor in the lung so that the inhibitor exhibits effects on a pulmonary disease. However, particularly, high-dose administration of an HMG-CoA reductase inhibitor may raise concerns about severe side effects such as rhabdomyolysis.

WO-A-00/ 48626 discloses pharmaceutical aerosol formulations comprising a HMG-CoA reductase inhibitor, in particular statins, for use in the treatment of asthma and pulmonary hypertension. It differs from the subject-matter of the present application in that it does not disclose the use of biocompatible polymer nanoparticles that contain the active agent nor the specific HMG-CoA reductase inhibitor pitavastatin.
Non-Patent Document 1: GINA Guideline, 2006
Non-Patent Document 2: GOLD Guideline, 2006
Non-Patent Document 3: Walter N., et al., Proc. Am. Thorac. Soc., Vol. 3, 330-338, 2006
Non-Patent Document 4: Goodman & Gilman, Pharmacological Basis of Therapeutics, 10th ed., McGraw hill, 2001
Non-Patent Document 5: Soyseth V., et al., Eur. Respir. J., 29, 279-283, 2007
Non-Patent Document 6: Mancini GBJ, et al., J. Am. Coll. Cardiol., 47, 2554-2560, 2006
Non-Patent Document 7: Hothersall E., et al., Thorax 61, 729-734, 2006

### Disclosure of the Invention

### Problems to be Solved by the Invention

An object of the present invention is to provide an excellent pulmonary disease therapeutic drug exhibiting high efficacy and reduced side effects.

### Means for Solving the Problems

The present inventors have conducted extensive studies for applying an HMG-CoA reductase inhibitor to a pulmonary disease therapeutic drug, and as a result have found that when an HMG-CoA reductase inhibitor is incorporated into biocompatible polymer nanoparticles, and the nanoparticles are administered directly to a main lesion site of pulmonary disease (i.e., bronchiole to alveoli), the nanoparticles exhibit an excellent pulmonary disease therapeutic effect at a low dose. The present invention has been accomplished on the basis of this finding.

Accordingly, the present invention provides a pulmonary disease therapeutic drug designed for intratracheal administration, comprising biocompatible polymer nanoparticles containing an HMG-CoA reductase inhibitor.
The present invention also provides use of biocompatible polymer nanoparticles containing an HMG-CoA reductase inhibitor for producing a pulmonary disease therapeutic drug designed for intratracheal administration.
The present invention also provides biocompatible polymer nanoparticles, containing an HMG-CoA reductase inhibitor, for use in the treatment of a pulmonary disease through intratracheal administration.
The present invention also provides a method for treatment of a pulmonary disease, comprising intratracheally administering an effective amount of biocompatible polymer nanoparticles containing an HMG-CoA reductase inhibitor.

### Effects of the Invention

According to the present invention, since biocompatible polymer nanoparticles containing an HMG-CoA reductase inhibitor can be administered directly to the lung by a simple procedure (e.g., inhalation), the nanoparticles are effectively transferred to a lesion site of pulmonary disease, and are accumulated in large amounts at the lesion site. Thus, the present invention provides a pulmonary disease therapeutic drug which exhibits higher efficacy at low dose and causes fewer side effects, as compared with the case where the HMG-CoA reductase inhibitor is orally administered for the purpose of the treatment of hyperlipidemia.

### Brief Description of the Drawings

[Fig. 1]

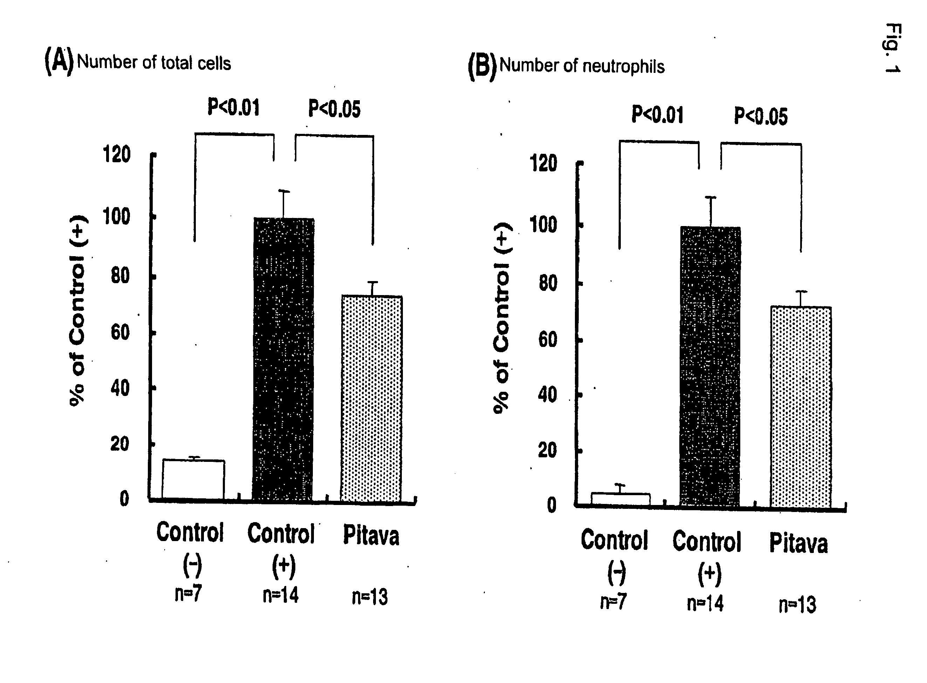

   Fig. 1 shows the effect of intratracheal administration of pitavastatin-calcium-incorporated PLGA nanoparticles on the number of cells contained in bronchoalveolar lavage fluid recovered from LPS-induced acute lung injury mice.
[Fig. 2]

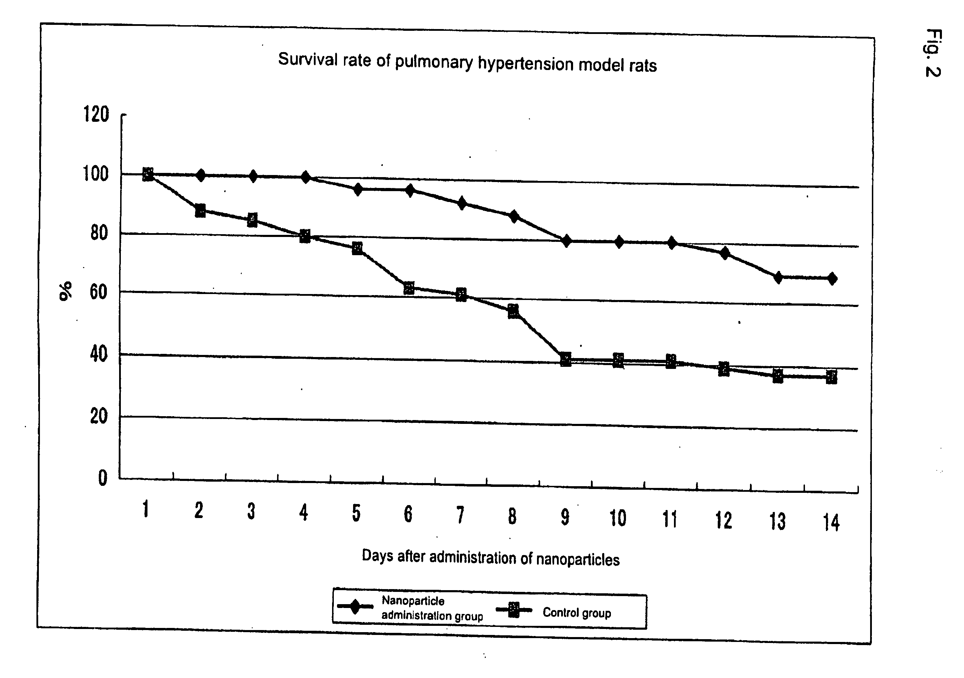

   Fig. 2 shows the survival rate of monocrotaline-induced severe pulmonary hypertensive rats after intratracheal administration of pitavastatin-calcium-incorporated PLGA nanoparticles.

### Best Modes for Carrying Out the Invention

The pulmonary disease therapeutic drug designed for intratracheal administration of the present invention comprises biocompatible polymer nanoparticles containing the HMG-CoA reductase inhibitor pitavastatin. The active ingredient of the drug is an HMG-CoA reductase inhibitor.
The HMG-CoA reductase inhibitor employed in the present invention encompasses the so-called statin compound pitavastatin which exhibits cholesterol synthesis inhibitory activity and is known as therapeutic agent for hyperlipidemia.

The preferred HMG-CoA reductase inhibitor is pitavastatin (Japanese Patent No. 2569746, US Patent Nos. 5102888 and 5856336, European Patent No. 304063), and salts thereof. Examples of salts of the HMG-CoA reductase inhibitor include alkali metal salts, alkaline earth metal salts, ammonium salts, and alkylammonium salts.

The salt of the HMG-CoA reductase inhibitor is particularly preferably a calcium salt or a sodium salt.

In the pulmonary disease therapeutic drug of the present invention, the HMG-CoA reductase inhibitor content of nanoparticles is preferably 0.001 to 20 wt.%, more preferably 0.005 to 20 wt.%, much more preferably 0.01 to 20 wt.%, particularly preferably 0.05 to 15 wt.%, from the viewpoint of effective drug delivery to a lesion site of the lung. As used herein, the expression "nanoparticles containing an HMG-CoA reductase inhibitor" encompasses both the case where the HMG-CoA reductase inhibitor is contained in the nanoparticles, and the case where the HMG-CoA reductase inhibitor is adsorbed on the surfaces of the nanoparticles.

Examples of the biocompatible polymer which forms nanoparticles include polylactic acid, polyglycolic acid, polyaspartic acid, lactic acid-glycolic acid copolymers, aspartic acid-lactic acid-glycolic acid copolymers, polyamide, polycarbonate, polyalkylene (e.g., polyethylene), polypropylene, polyethylene glycol, polyethylene oxide, polyethylene terephthalate, polyvinyl compounds (e.g., polyvinyl alcohol, polyvinyl ether, and polyvinyl ester), acrylic acid-methacrylic acid polymers, cellulose and other polysaccharides, peptides, proteins, and copolymers or mixtures thereof. Of these, polylactic acid, polyglycolic acid, a lactic acid-glycolic acid copolymer (PLGA), and a block copolymer of any of these polymers and polyethylene glycol (PEG) are more preferred, with a block copolymer of a lactic acid-glycolic acid copolymer (PLGA) and polyethylene glycol (PEG) (PEG-modified PLGA, peg-PLGA) being particularly preferred. Any of the aforementioned biocompatible polymers can contain therein an HMG-CoA reductase inhibitor, and the drug-contained polymer can be stored for a long period of time while the efficacy of the drug is maintained. Conceivably, by virtue of degradation of the biocompatible polymer by an enzyme in vivo, sustained release of the HMG-CoA reductase inhibitor can be attained in the lung over several hours to several tens of hours.

The biocompatible polymer preferably has a molecular weight of 5,000 to 200,000, particularly preferably 15,000 to 25,000. When the biocompatible polymer is a lactic acid-glycolic acid copolymer (PLGA), the ratio by mole of lactic acid to glycolic acid may be 1 : 99 to 99 : 1, but is preferably 1 : 0.333. A PLGA having a lactic acid or glycolic acid content of 25 wt.% to 65 wt.% is preferably employed, since such a PLGA is amorphous and can be dissolved in an organic solvent such as acetone.

The nanoparticles employed in the present invention preferably have a particle size of 30 nm to 10 µm, particularly preferably 100 nm to 5 µm, from the viewpoints of effective drug delivery to a lesion site of the lung and effective incorporation of an HMG-CoA reductase inhibitor. The particle size of the nanoparticles can be measured by means of Coulter Counter N4 PLUS (product of Beckman Coulter Inc.).

The nanoparticles employed in the present invention may be produced through a method described in, for example, Journal of the Society of Powder Technology 42 (11), 765-772 (2005), JP-A-2003-275281, JP-A-2004-262810, or JP-A-2006-321763.

Next will be described an example of production of the nanoparticles of the present invention by the method based on diffusion of emulsion solvent in purified water.
A PLGA is dissolved in an organic solvent such as acetone, to thereby prepare a polymer solution. The polymer solution is mixed with an HMG-CoA reductase inhibitor or an aqueous solution thereof. The resultant mixture is added dropwise to an aqueous polyvinyl alcohol (PVA) solution, purified water, etc. under stirring, to thereby prepare an emulsion. The organic solvent (e.g., acetone) is removed by evaporation, to thereby form a suspension of PLGA nanoparticles, and the suspension is centrifuged. The thus-precipitated PLGA nanoparticles are recovered and resuspended in purified water, and washed so that excess PVA which has not been adsorbed on the surface of PLGA nanoparticles is removed, followed by lyophilization, to thereby form powder. Alternatively, the suspension of PLGA nanoparticles may be lyophilized without resuspending, to thereby form powder.

The nanoparticles of the present invention can form a composite with higher-order structure, and encompass the thus-formed nanoparticle composite. Such a nanoparticle composite can be produced by uniformly mixing a sugar alcohol with a liquid containing nanoparticles produced by, for example, any of the aforementioned methods, and lyophilizing the resultant mixture. Examples of the sugar alcohol include mannitol, trehalose, sorbitol, erythritol, maltitol, and xylitol. The amount of the sugar alcohol added is preferably 0.001 to 1 wt.%, particularly preferably 0.01 to 0.1 wt.%, on the basis of the entirety of the nanoparticle-containing liquid.

Preferably, when in use, the nanoparticles of the present invention are contained in an aqueous solution such as saline (Japanese Pharmacopoeia) (pH = 6.0) or purified water (pH = 6.8). The nanoparticle-containing liquid preferably contains a dispersant such as polyvinyl alcohol or polyethylene glycol. The nanoparticle concentration of the nanoparticle-containing liquid is preferably 0.1 to 20 wt.%, particularly preferably 1 to 10 wt.%, from the viewpoint of prevention of aggregation of particles. The dispersant concentration of the nanoparticle-containing liquid is preferably 0.1 to 20 wt.%, particularly preferably 1 to 10 wt.%, from the viewpoint of effective dispersion of the nanoparticles.

The daily dose of the nanoparticles of the present invention (as reduced to HMG-CoA reductase inhibitor), which is appropriately determined in consideration of the type of disease and symptoms, is 0.001 to 100 mg, preferably 0.01 to 50 mg, more preferably 0.01 to 30 mg, much more preferably 0.1 to 10 mg. Particularly when the HMG-CoA reductase inhibitor is pitavastatin or a salt thereof, the daily dose thereof is preferably 0.001 to 50 mg, more preferably 0.01 to 30 mg.

Administration of the drug of the present invention to the lung is preferably carried out by means of, for example, an inhaler or a nebulizer. The frequency of administration may be once to thrice a day in the case of high-frequency dose, or may be once every two or three days or once a week in the case of low-frequency dose.

When the drug of the present invention is administered directly to the lung (e.g., bronchiole to alveoli), the drug is delivered to a lesion site of the lung, and the HMG-CoA reductase inhibitor is released over a long period of time. Therefore, a low dose of the drug realizes safe treatment of a pulmonary disease. Examples of the pulmonary disease to be treated by the drug include pulmonary hypertension, chronic obstructive pulmonary disease, pulmonary fibrosis, acute respiratory distress syndrome, bronchial asthma, inflammatory pulmonary disease, pneumonia, and bronchitis. The drug is particularly useful for the treatment of pulmonary hypertension.
When adrenocortical steroid is employed for the treatment of a pulmonary disease in combination with the therapeutic drug of the present invention, the dose of adrenocortical steroid can be reduced, which leads to reduction of side effects of the steroid.

### Examples

The present invention will next be described in more detail by way of examples, which should not be construed as limiting the invention thereto.

### Example 1

### Method for preparing pitavastatin-calcium-salt-incorporated PLGA nanoparticles

Incorporation of a calcium salt of pitavastatin (pitavastatin calcium) (Japanese Patent No. 2569746, US Patent Nos. 5102888 and 5856336, European Patent No. 304063) into PLGA nanoparticles was carried out according to a previously reported method based on diffusion of emulsion solvent in purified water (Journal of the Society of Powder Technology 42, 765-772 (2005)).

A lactic acid-glycolic acid copolymer (PLGA, molecular weight: 20,000, ratio of lactic acid/glycolic acid: 75/25) (1 g) and pitavastatin calcium (0.025 g) were dissolved in acetone (40 mL), and ethanol (20 mL) was added to the solution, to thereby prepare a polymer solution. The polymer solution was added dropwise to an aqueous PVA solution (an aqueous solution (100 mL) containing PVA (0.5 g)) stirred at 400 rpm by means of a stirrer, to thereby form an emulsion. The organic solvent was removed from the emulsion by evaporation with stirring under reduced pressure at 40°C for one hour, followed by filtration with a membrane filter. Thereafter, the filtrate was lyophilized, to thereby produce PLGA nanoparticles of interest in the form of powder.
The PLGA nanoparticles were found to have a pitavastatin calcium content of 1.3 wt.%.
As a control, PLGA nanoparticles containing no pitavastatin calcium were prepared in the same manner as described above, except that pitavastatin calcium was not added.

### Example 2

### Method for preparing pitavastatin-calcium-salt-incorporated PEG-modified PLGA nanoparticles

PEG-modified PLGA (peg-PLGA) (2 g) and pitavastatin calcium (0.1 g) were dissolved in acetone (20 mL), and ethanol (10 mL) was added to the solution, to thereby prepare a polymer solution.
The polymer solution was added dropwise to purified water (50 mL) stirred at 400 rpm and at 40°C.
The organic solvent was removed from the emulsion by evaporation under reduced pressure at 40°C over two hours, and then the suspension was filtered with a membrane filter having a pore size of 32 µm, so as to remove aggregated nanoparticles.
The filtrate was employed as is in Test Example 2. The nanoparticle-containing liquid was found to have a pitavastatin calcium content of 0.0998 wt.%.

### Test Example 1

### Effect of intratracheal administration of pitavastatin-calcium-incorporated PLGA nanoparticles on inflammatory cells in LPS-induced acute lung injury model

### Test method:

Male BALB/c mice purchased from Charles River Laboratories Japan Inc. (eight weeks old upon test) were preliminarily reared in a rearing chamber (temperature: 21 ± 2°C, humidity: 50 ± 20%, light period: 7:00 to 19:00) under conditions where feed and water were fed ad libitum. The thus-reared mice were employed for the test.
For the test, mice were divided into the following three groups: a group of mice without inhalation exposure to LPS (lipopolysaccharide) (Control (-) group, n = 7); a group of mice with administration of non-pitavastatin-calcium-incorporated PLGA nanoparticles and then inhalation exposure to LPS (Control (+) group, n = 14); and a group of mice with administration of pitavastatin-calcium-incorporated PLGA nanoparticles and then inhalation exposure to LPS (Pitava group, n = 13).

Each of the mice of Control (+) group and Pitava group was anesthetized through intraperitoneal injection of pentobarbital sodium (50 mg/10 mL/kg), and the cervix of the mouse was incised and the airway thereof was exposed. Non-pitavastatin-calcium-incorporated PLGA nanoparticles (for the mice of Control (+) group) or pitavastatin-calcium-incorporated PLGA nanoparticles (for the mice of Pitava group) were suspended in saline under visual observation, and the resultant suspension (50 µL) was intratracheally administered together with air (200 µL) by a 27G injection needle. After intratracheal administration, the cervix was sutured, and mice aroused from anesthesia were sequentially returned to the rearing cage.
The dose (by weight) of administration of each type of the PLGA nanoparticles was 15 µg/body (pitavastatin calcium content: 0.2 µg/body for Pitava group).
The nanoparticle liquid to be administered was reconstituted upon use by suspending the nanoparticles in saline, followed by sonication for 30 seconds by means of an ultrasonic homogenizer.

Twenty-four hours after intratracheal administration, each of the PLGA-nanoparticles-administered mice was transferred to a cage made of acrylic material and having inner dimensions of 26 cm (W) x 26 cm (D) x 10 cm (H), and LPS (Sigma) (30 µg/mL) nebulized by means of an ultrasonic nebulizer (Omron Corporation) was fed to the cage for inhalation exposure of the mouse to LPS. The inhalation exposure was continued for 30 minutes, and the thus-exposed mouse was returned to the rearing cage.
Four hours after initiation of inhalation exposure to LPS, each of the test mice was anesthetized through intraperitoneal injection of pentobarbital sodium (50 mg/10 mL/kg). The abdominal aorta of the mouse was dissected for bleeding to death. Thereafter, the posterior cervix of the mouse was incised, and a polyethylene tube having an outer diameter of 1.2 mm (SP55, product of Natsume Seisakusho Co., Ltd.) was fixed to the bronchus. Bronchoalveolar lavage was carried out by repeating thrice a process including injection and recovery of phosphate-buffered saline (PBS) (1 mL). The thus-recovered bronchoalveolar lavage fluid (BALF) was subjected to centrifugation at 1,000 rpm and 4°C for 10 minutes, and the collected migratory cells were resuspended in PBS (200 µL). The total number of the cells and the number of neutrophils were counted by means of an automated hematology analyzer (XT-2000i, Sysmex).

### Test results:

Table 1 and Fig. 1 show the results (average value (represented by percentage with respect to the average (taken as 100) of data of Control (+) group), and standard error).

**[Table 1]**

| | | n | Average value | Standard error |
|---|---|---|---|---|
| Total number of cells | Control (-) group | 7 | 14.6 | 3.6 |
| | Control (+) group | 14 | 100 | 8.8 |
| | Pitava group | 13 | 74.0 | 5.4 |
| Number of neutrophils | Control (-) group | 7 | 4.7 | 3.1 |
| | Control (+) group | 14 | 100 | 9.8 |
| | Pitava group | 13 | 72.6 | 5.8 |

Intratracheal administration of pitavastatin-calcium-incorporated PLGA nanoparticles significantly inhibited migration of inflammatory cells (p < 0.05). The data indicated that migration of inflammatory cells is inhibited not by PLGA nanoparticles themselves, but by pitavastatin-calcium-incorporated PLGA nanoparticles.
In a manner similar to that described above, another test was carried out by use of a solution prepared by dissolving pitavastatin calcium (i.e., non-polymer-incorporated form) (0.2 µg/body, 2 µg/body, or 20 µg/body) in saline (50 µL). However, intratracheal administration of any of the aforementioned three doses of pitavastatin calcium did not exhibit the effect of inhibiting migration of inflammatory cells (a group of non-administration of pitavastatin calcium: n = 8, and a group of administration of pitavastatin calcium (any of the aforementioned doses): n = 8).
In the aforementioned tests, local administration of pitavastatin-calcium-incorporated PLGA nanoparticles to the lung inhibited LPS-induced inflammatory response. In this case, the dose of pitavastatin calcium required for inhibiting inflammatory response was considerably reduced, as compared with the case where pitavastatin calcium was administered without being incorporated in PLGA nanoparticles. This indicates that pitavastatin-calcium-incorporated PLGA nanoparticles are useful for the treatment of a pulmonary disease associated with inflammation.

### Test Example 2

### Test on treatment of pulmonary hypertension by intratracheal administration of pitavastatin-calcium-incorporated PLGA nanoparticles

### Test method:

Monocrotaline (MCT) was subcutaneously injected (60 mg/kg body weight) to male SD rats (seven weeks old, 250 to 300 g), to thereby prepare MCT-induced pulmonary hypertensive rats (severe pulmonary hypertension is established three weeks after MCT administration).
The rats were divided into the following two groups: (1) a first group (i.e., a group of rats with administration of pitavastatin-calcium-incorporated PEG-modified PLGA nanoparticles (nanoparticle administration group, n = 26)); and (2) a second group (i.e., a group of rats with administration of PBS (control) (control group, n = 41)).
On day 21 after administration of MCT, the anterior cervix of each rat was incised. A liquid containing pitavastatin-calcium-incorporated PEG-modified PLGA nanoparticles (produced in Example 2 above) (100 µL) and air (100 µL) were intratracheally administered to each of the rats of the first group, and PBS (100 µL) and air (100 µL) were intratracheally administered to each of the rats of the second group. The pitavastatin-calcium-incorporated PEG-modified PLGA nanoparticles were found to contain pitavastatin calcium in an amount of 100 µg/body.
Survival of the rats was observed for a period of 14 days after administration of the nanoparticles.

### Test results:

As shown in Table 2 and Fig. 2, in the control group, the survival rate of the rats (14 days after administration) was reduced to 37%, whereas in the nanoparticle administration group, the survival rate (14 days after administration) of the rats was 69%, which was significantly improved with respect to that of the control group (p < 0.01).

**[Table 2]**

| | Number of rats employed | Number of rats survived 14 days after administration | Survival rate |
|---|---|---|---|
| Control group | 41 | 15 | 37% |
| Nanoparticle administration group | 26 | 18 | 69% |

The test results correspond to the case where a very low dose of pitavastatin calcium (i.e., 100 µg) is administered once for the treatment of pulmonary hypertension. The above data indicate that intratracheal administration of pitavastatin-calcium-incorporated PLGA nanoparticles is very effective.
In the case where an HMG-CoA reductase inhibitor is orally administered for the treatment of hyperlipidemia, atorvastatin calcium salt requires a high dose (10 to 80 mg/day), pitavastatin calcium salt requires a low dose (1 to 4 mg/day). The data obtained in Test Examples 1 and 2 indicate that intratracheal administration of HMG-CoA-reductase-inhibitor-incorporated biocompatible polymer nanoparticles exhibits the effect of suppressing a pulmonary disease, even when the dose of the HMG-CoA reductase inhibitor is lower than that in the case where the inhibitor is used for the treatment of hyperlipidemia.

## Claims

1. A drug designed for intratracheal administration for use in the therapy of pulmonary disease, said drug comprising biocompatible polymer nanoparticles containing pitavastatin or a salt thereof as an HMG-CoA reductase inhibitor,
wherein said biocompatible polymer is a lactic acid-glycolic acid copolymer, or a block copolymer of lactic acid-glycolic acid copolymer and polyethylene glycol.

2. The drug according to claim 1, wherein the pulmonary disease is pulmonary hypertension, chronic obstructive pulmonary disease, pulmonary fibrosis, acute respiratory distress syndrome, bronchial asthma, inflammatory pulmonary disease, pneumonia, or bronchitis.

3. Biocompatible polymer nanoparticles containing pitavastatin or a salt thereof as an HMG-CoA reductase inhibitor,
for use in the treatment of a pulmonary disease through intratracheal administration;
wherein said biocompatible polymer is a lactic acid-glycolic acid copolymer, or a block copolymer of lactic acid-glycolic acid copolymer and polyethylene glycol.

4. The biocompatible polymer nanoparticles according to claim 3, wherein the pulmonary disease is pulmonary hypertension, chronic obstructive pulmonary disease, pulmonary fibrosis, acute respiratory distress syndrome, bronchial asthma, inflammatory pulmonary disease, pneumonia, or bronchitis.

## Patentansprüche

1. Arzneimittel für die intratracheale Verabreichung zur Verwendung bei der Behandlung einer Lungenerkrankung, wobei das Arzneimittel biokompatible polymere Nanopartikel enthält, die Pitavastatin oder ein Salz desselben als HMG-CoA-Reduktase-Inhibitor enthalten, wobei das biokompatible Polymer ein Milchsäure-Glykolsäure-Copolymer oder ein Block-Copolymer aus einem Milchsäure-Glykolsäure-Copolymer und Polyethylenglykol ist.

2. Das Arzneimittel gemäß Anspruch 1, wobei die Lungenerkrankung pulmonale Hypertonie, chronische obstruktive Lungenerkrankung, Lungenfibrose, Acute Respiratory Distress Syndrome, Lungenasthma, entzündliche Lungenerkrankung, Lungenentzündung oder Bronchitis ist.

3. Biokompatible polymere Nanopartikel enthaltend Pitavastatin oder ein Salz desselben als HMG-CoA-Reduktase-Inhibitor zur Verwendung bei der Behandlung einer Lungenerkrankung mittels intratrachealer Verabreichung, wobei das biokompatible Polymer ein Milchsäure-Glykolsäure-Copolymer oder ein Block-Copolymer aus einem Milchsäure-Glykolsäure-Copolymer und Polyethylenglykol ist.

4. Die biokompatible polymeren Nanopartikel gemäß Anspruch 3, worin die Lungenerkrankung pulmonale Hypertonie, chronische obstruktive Lungenerkrankung, Lungenfibrose, Acute Respiratory Distress Syndrome, Lungenasthma, entzündliche Lungenerkrankung, Lungenentzündung oder Bronchitis ist.

## Revendications

1. Médicament conçu pour être administré par voie intra-trachéale, pour utilisation dans le traitement d'une maladie pulmonaire, ledit médicament comprenant des nanoparticules polymères biocompatibles contenant de la pitavastatine ou un sel de celle-ci en tant qu'inhibiteur de HMG-CoA réductase, dans lequel ledit polymère biocompatible est un copolymère d'acide lactique/acide glycolique, ou un copolymère séquencé de copolymère d'acide lactique/acide glycolique et de polyéthylèneglycol.

2. Médicament selon la revendication 1, dans lequel la maladie pulmonaire est l'hypertension pulmonaire, la broncho-pneumopathie chronique obstructive, la fibrose pulmonaire, le syndrome de détresse respiratoire aiguë, l'asthme bronchique, une maladie pulmonaire inflammatoire, une pneumonie, ou une bronchite.

3. Nanoparticules polymères biocompatibles contenant de la pitavastatine ou un sel de celle-ci en tant qu'inhibiteur de HMG-CoA réductase, pour utilisation dans le traitement d'une maladie pulmonaire par administration par voie intra-trachéale ; dans lesquelles ledit polymère biocompatible est un copolymère d'acide lactique/acide glycolique, ou un copolymère séquencé de copolymère d'acide lactique/acide glycolique et de polyéthylèneglycol.

4. Nanoparticules polymères biocompatibles selon la revendication 3, dans lesquelles la maladie pulmonaire est l'hypertension pulmonaire, la broncho-pneumopathie chronique obstructive, la fibrose pulmonaire, le syndrome de détresse respiratoire aiguë, l'asthme bronchique, une maladie pulmonaire inflammatoire, une pneumonie, ou une bronchite.
